# EUROPEAN PATENT SPECIFICATION

(11) **EP 3 563 760 B1**
(45) Date of publication and mention of the grant of the patent: **28.02.2024**
(21) Application number: 19168010.7
(22) Date of filing: 09.04.2019
(51) Int. Cl.: A61B 5/00, A61B 5/24, H05K 1/02, H05K 1/11

(54) **POLYMER HOLDER FOR A BIOSIGNAL PROCESSING DEVICE**
POLYMERHALTER FÜR EIN BIOSIGNALVERARBEITUNGSGERÄT
SUPPORT EN POLYMERE POUR UN DISPOSITIF DE TRAITEMENT DE SIGNAL BIOLOGIQUE

(30) Priority: 30.04.2018 US 201815966785
(43) Date of publication of application: 06.11.2019
(73) Proprietor: Bittium Biosignals Oy, 70800 Kuopio (FI)
(72) Inventor: Myllykangas, Juha, 70800 Kuopio (FI); Nikula, Arto, 90590 Oulu (FI); Remes, Arto, 70800 Kuopio (FI)
(74) Representative: Kolster Oy Ab

(56) References cited:
- EP-A1- 2 468 181
- EP-A2- 1 559 335
- WO-A1-2015/127218
- WO-A1-2015/189476
- WO-A2-2014/057083
- US-A1- 2012 035 426

## Description

### Field

The invention relates to a polymer holder for a biosignal processing device, an electrode system and manufacturing and handling methods of the polymer holder.

### Background

An electronic device, which measures biosignals such as ECG (ElectroCardioGram) and EEG (ElectroEncephaloGram), must be well contacted with the electrodes that are in contact with the body and mechanically reliably fixed to its support. The electronic device should also be easily removable from the support. A good attachment with easy detachment has not yet been discovered. Document EP2468191 presents a single radio-transparent connector for a multifunctional reference patch. Document WO2015127218 presents a separable monitoring device and a method. Document EP1559335 presents a garment, in particular a shirt. Document WO2015189476 presents an electrode band for sensing a bio-electrical signal. Document WO2104057083 presents a monitoring device. Document US20120035426 discloses a docking station to be attached to a shirt and comprising a housing for receiving a portable transmitting unit. The portable transmitting unit is detachably removable from the housing thanks to a latching mechanism. Hence, there is a need for improvement.

### Brief description

The invention is defined by the independent claims. Preferred embodiments are defined in the dependent claims.

### List of drawings

Example embodiments of the present invention are described below, by way of example only, with reference to the accompanying drawings, in which
Figures 1A and 1B illustrate examples of polymer holders;
Figure 2 illustrates an example of an electrically conductive contact structure within the polymer holder;
Figure 3 illustrates an example of the polymer holder with a second aperture and a piston for moving a biosignal processing device fromward the polymer holder;
Figure 4 illustrates an example of the polymer holder with the second aperture directed to a different direction from that of a first aperture;
Figure 5 illustrates an example of the polymer holder with a first part and a second part which are coupled with a hinge;
Figure 6 illustrates an example of the second part with the hinged piston;
Figure 7 illustrates an example of the second part with elastic material therein;
Figures 8A and 8B illustrate an example of a lever for pushing the biosignal processing device outward from the polymer holder;
Figure 9 illustrates an example of a seal round the electrically conductive contact structure;
Figure 10 illustrates an example where the biosignal processing device has at least one processor and at least one memory;
Figure 11 illustrates an example of a flow chart of a manufacturing method; and
Figure 12 illustrates of an example of a flow chart of a handling method of the polymer holder.

### Description of embodiments

The following embodiments are only examples. Although the specification may refer to "an" embodiment in several locations, this does not necessarily mean that each such reference is to the same embodiment(s), or that the feature only applies to a single embodiment. Single features of different embodiments may also be combined to provide other embodiments. Furthermore, words "comprising" and "including" should be understood as not limiting the described embodiments to consist of only those features that have been mentioned and such embodiments may also contain features/structures that have not been specifically mentioned. All combinations of the embodiments are considered possible if their combination does not lead to structural or logical contradiction.

It should be noted that while Figures illustrate various embodiments, they are simplified diagrams that only show some structures and/or functional entities. The connections shown in the Figures may refer to logical or physical connections. It is apparent to a person skilled in the art that the described apparatus may also comprise other functions and structures than those described in Figures and text. It should be appreciated that details of some features are irrelevant to the actual invention. Therefore, they need not be discussed in more detail here.

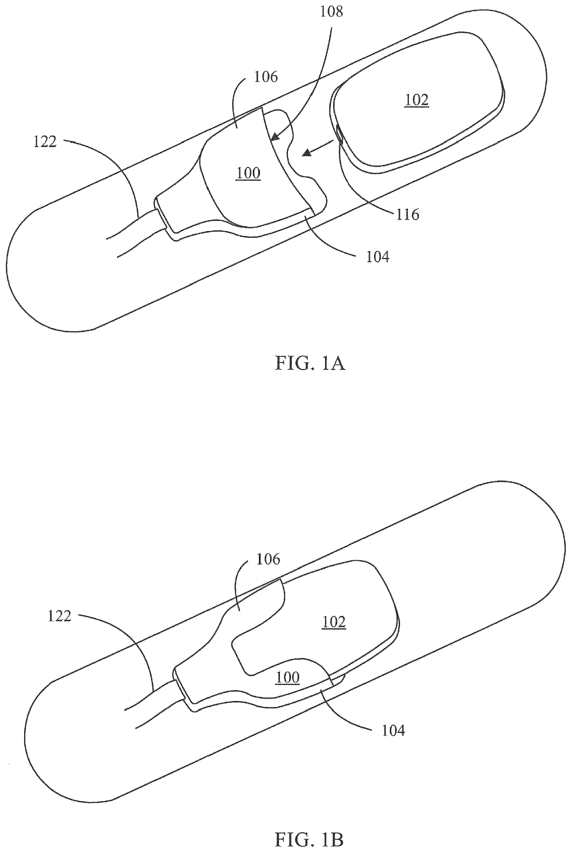

Figure 1A and 1B illustrate example of polymer holders 100 for a biosignal processing device 102. In Figure 1B, the polymer holder 100 has a slit in the front part whereas the polymer holder 100 of Figure 1A does not have it. The name refers to the fact that the polymer holder 100 is made of polymer such as plastic. The biosignal processing device 102 may be an electronic device which may convert an analog biosignal it receives to a digital biosignal. The biosignal processing device 102 may also filter the biosignal in the analog or in the digital form. Additionally or alternatively, the biosignal processing device 102 may perform data processing of the biosignal, and it may also store data of the biosignal and/or a result of its processing. The biosignal may be related to body movement, body temperature, heart rate variability, electrocardiogram, electromyogram, electroencephalogram or the like for example.

In Figure 1A the biosignal processing device 102 is outside the polymer holder 100. The arrow in Figure 1A illustrates the feature that the biosignal processing device 102 can be inserted in the polymer holder 100. In Figure 1B the biosignal processing device 102 is inserted in the polymer holder 100.

A wall 104 of the polymer holder 100 forms a pocket 106, and the wall 104 follows an outer contour of the biosignal processing device 102. The pocket 106 is a free space or volume into which a part of the biosignal processing device 102 fits accurately. A degree of precision with which the surfaces of the biosignal processing device 102 and the polymer holder 100 are adapted to each other may be high enough to enable operation with one hand or without seeing the actual movement of processing device 102. A friction between an outer surface of the biosignal processing device 102 and an inner surface of the polymer holder 100 may keep the biosignal processing device 102 in the polymer holder 100 even under accelerations caused by sport activities or in upside down positions. The fit between the biosignal processing device 102 and the polymer holder 100 may be rather tight resulting in a suitable friction and suction force. Polymer material of the holder 100 is also slightly flexible and even stretchable which enables to achieve a suitable tightness and friction and suction force between the polymer holder 100 and the biosignal processing device 102. The pocket 106 has the wall 104 round the biosignal processing device 102 in a continuous hemispherical manner, which is more secure and efficient than a wall that has a shape of band round the biosignal processing device 102, for example. Still, the hemispherical wall 104 allows easy removal of the biosignal processing device 102.

The polymer holder 100 has a first aperture 108 for inserting the biosignal processing device 102 into the pocket 106 and removing the biosignal processing device 102 from the pocket 106. The size of the aperture 108 may vary but a size and a shape of a cross section of the aperture 108 and the biosignal processing device 102 are about the same.

However in an embodiment, the aperture first 108 may be larger because there may be a clearance between the biosignal processing device 102 and the polymer holder 100, the clearance allowing a free play between the biosignal processing device 102 and the polymer holder 100 at the first aperture 108. In an embodiment, the clearance may be less than 1 mm. In an embodiment, the clearance may be less than 0.1 mm. In any embodiment, the clearance may be less than 0.01 mm. In any embodiment having the clearance, the clearance may be larger than 0.001 mm.

In an embodiment, the cross section of the first aperture 108 and/or the pocket 106 may be a slightly smaller than that of the biosignal processing device 102, because the polymer holder 100 may stretch. In this manner, the friction between the polymer holder 100 and the biosignal processing device 102 may be made stronger.

The polymer holder 100 includes an electrically conductive contact structure 110 at a rear section 114 of the polymer holder 100 opposite to the first aperture 108. The electrically conductive contact structure 110 may be in a wired electric contact with electrodes 112, which are configured to receive the at least one biosignal. The biosignal may be generated by a human or animal. The electrically conductive contact structure 110 may be at least partly attached to the wall 104, directly or indirectly to hold structure 100 in place. The electrically conductive contact structure 110 is configured to connect electrically with a counterpart 116 of the biosignal processing device 102 in response to an insert of the biosignal processing device 102 into the pocket 106. The electrically conductive contact structure 110 is also configured to connect mechanically with the counterpart 116 which may increase friction and thus permanence, immobility and/or stability of the biosignal processing device 102 partly within the polymer holder 100. By having a proper wall 104 around the biosignal processing device 102 in a form of the pocket 106 and a good electric contact inside the pocket 106 makes the biosignal processing device 102 to stay in the pocket 106 effectively even in rapid movements. The biosignal processing device 102 is also easy to take out from the pocket 106, because it is only partly therein.

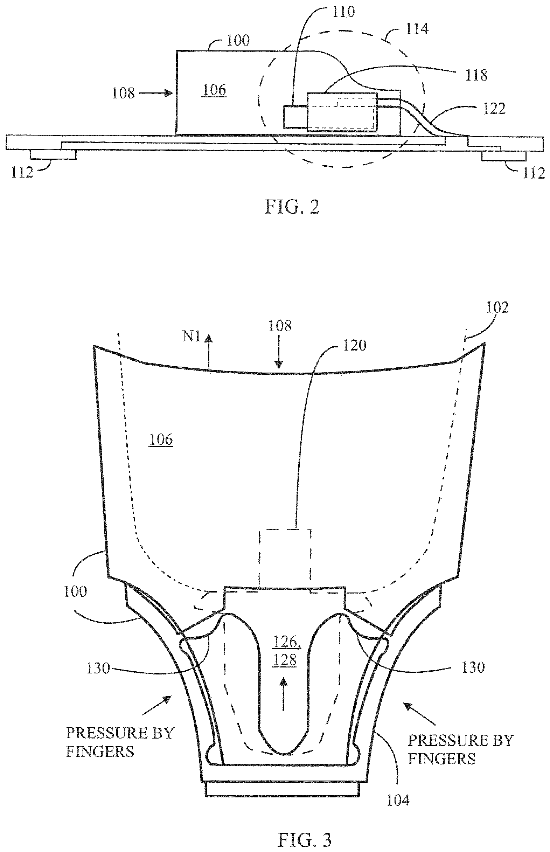

In an embodiment an example of which is illustrated in Figure 2, the electrically conductive contact structure 110 may comprise a male connector that extends at least partly inside the pocket 106. The male connector may connect with a female connector of the biosignal processing device 102 in response to the insert of the biosignal processing device 102 in the pocket 106.

In an embodiment, the electrically conductive contact structure 110 may comprise a female connector, and the female connector is configured to connect with a male connector of the biosignal processing device 102 in response to the insert of the biosignal processing device 102 in the pocket 106.

In an embodiment, the male connector may be a male USB connector (USB = Universal Serial Bus), and the female connector may be a female USB connector. In an embodiment, the male connector may be a male micro USB connector, and the female connector may be a female micro USB connector.

In an embodiment, the electrically conductive contact structure 110 is partly within a cover 118 of an elastic material. In an embodiment, the cover 118 may be molded, and the electrically conductive contact structure 110 may be left within the elastic material. In an embodiment, the molding process may be injection molding.

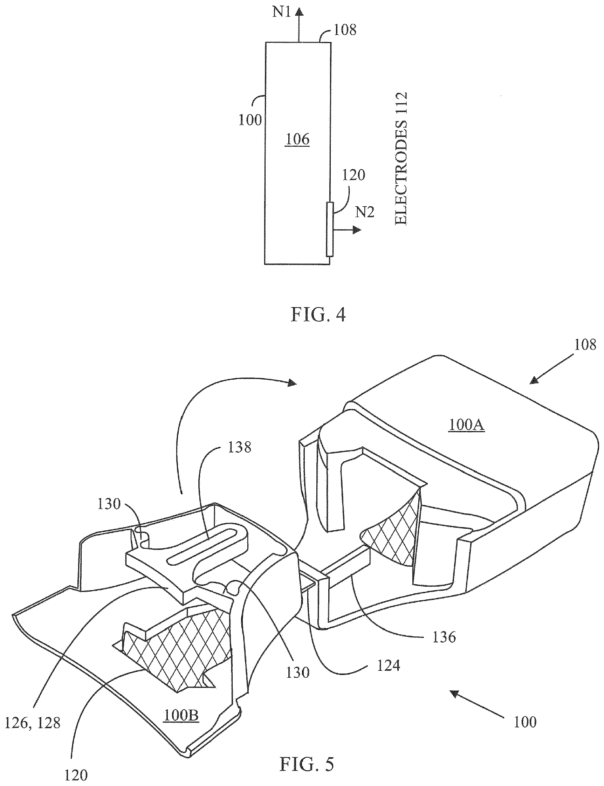

In an embodiment an example of which is illustrated in Figure 3, the polymer holder 100 may have a second aperture 120 at the rear section 114. A normal N2 of the second aperture 120 may be directed toward a direction perpendicular to a normal N1 of the first aperture 108, which is illustrated in Figure 4. The second aperture 120 may be configured to open toward the electrodes 112, which is also illustrated in Figure 4. The electrically conductive contact structure 110 may be inserted through the second aperture 120 into the polymer holder 100. The electrically conductive contact structure 110 may have the cover 118 while it is inserted through the second aperture 120. The electrically conductive contact structure 110 may be pig-tailed while inserting it through the second aperture 120 (see also Figures 1A, 1B and 2). A wire 122 of the pig-tail may connect the electrically conductive contact structure 110 with the electrodes 112.

In an embodiment an example of which is illustrated in Figure 5, the polymer holder 100 may comprise a first polymer part 100A, a second polymer part 100B and a flexible polymer hinge 124, which are of the same material combined together in a common molding process. The hatched parts in Figure 5 are holes. In an embodiment, the molding process may be an injection molding process. The hinge 124 allows about 180° rotation between the first polymer part 100A and the second polymer part 100B such that the first polymer part 100A and the second polymer part 100B overlap each other for forming the usable polymer holder 100.

In an embodiment, the first polymer part 100A and the second polymer part 100B may be formed separately.

According to the invention, as illustrated in Figures 3, 5 and 6, the polymer holder 100 comprises, at the rear section 114, a mechanism 126 which can cause a force to the biosignal processing device 102 directed toward the first aperture 108 in response to pressure against the polymer holder 100 at the rear section 114. The pressure against an outer surface of the polymer holder 100 may be caused by a user when he/she presses the polymer holder 100 by his/her fingers, for example. The force against and the movement of the biosignal processing device 102 with the help of the force provided by the mechanism 126 makes it easy to detach the biosignal processing device 102 from the pocket 106. The biosignal processing device 102 may be gripped with fingers from the upper part for lifting the biosignal processing device 102 and additionally the pressure against the polymer holder 100 results in a convenient removal of the biosignal processing device 102 from the pocket 106. In an embodiment, the mechanism 126 may move about 1 mm when a pressure is applied at the rear section 114. In an embodiment, the mechanism 126 may be configured to move about 2 mm when a pressure is applied at the rear section 114. The biosignal processing device 102 may be configured to move about the same length at the mechanism 126 when a pressure is applied at the rear section 114.

Figures 3, 5 and 6 show the mechanism 126 that comprises a piston 128 that can move toward and fromwards the first aperture 108. The mechanism 126 also comprises least one lever 130 hinged to the piston 128 at one end and to the polymer holder 100 at another end. A hinge 132 between the lever 130 and the polymer holder 100 may be made of the same material as the polymer holder 100. The hinge 134 between the lever 130 and the piston 128 may be made of the same material as the polymer holder 100. The common molding process enables this kind of hinging.

In an embodiment, the at least one lever 130 may be pushed forward and turn between the hinges 132, 134 in response to a pressure of the polymer holder 100 at the rear section 114. The lever 130 may turn between the hinges 132, 134. Then the lever 130 may move the piston 128 toward the first aperture 108 in response to the pressure. The piston 128 may then move the biosignal processing device 102 toward the first aperture 108.

In an embodiment an example of which is illustrated in Figure 5, the polymer holder 100 (second part 100B) may have a rail 136 extending longitudinally in a direction from the rear section 114 to the first aperture 108. The piston 128 may have a slit 138 into which the rail 136 may be matched for guiding the movement of the piston 128 toward and fromward the first aperture 108.

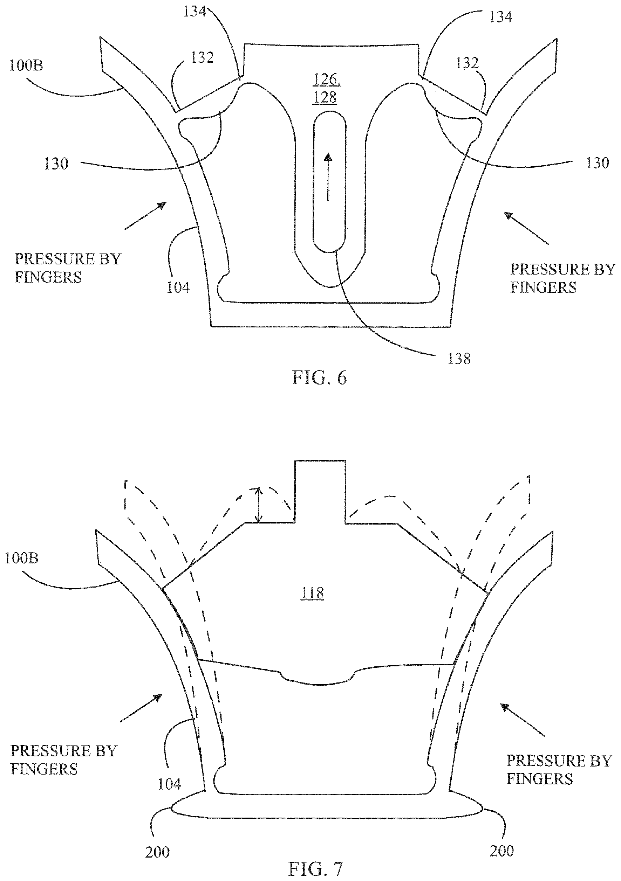

In an embodiment, the wall 104 of the polymer holder 100 may droop in response to the pressure. The mechanism 126 may then redirect the force of the pressure to a force toward the first aperture 108 in order to move the biosignal processing device 102 thereto. As can be seen in Figure 6 the wall 104 may easily bend at the hinges 132, 134.

In an embodiment an example of which is shown in Figure 7, the electrically conductive contact structure 110 may comprise elastic material as a cover 118 at the rear section 114. The pressure against the second part 100B of the polymer holder 100 may cause the elastic material of the cover 118 to reshape, push against and move the biosignal processing device 102 toward the first aperture 108 for enabling an easy removal. The dashed lines show the movement of the wall 104 of the second part 100B of the polymer holder 100 and the movement of elastic material of the cover 118 toward the first aperture 108. The elastic material of the cover 118 may revert to its original shape after the pressure ceases.

In an embodiment, the polymer holder 100 has extensions 200 at a farthest end from the first aperture 108 to ensure a good grip when the polymer holder 100 is squeezed.

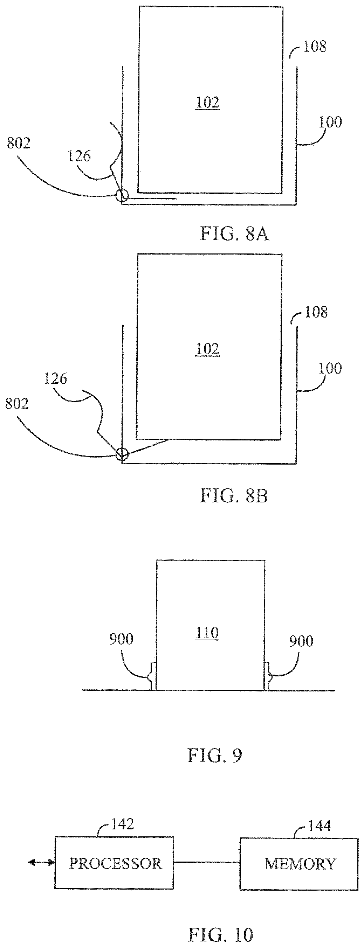

In an embodiment an example of which is shown in Figures 8A and 8B, the mechanism 126 may be a hand lifted lever 800 outside the polymer holder 100. The lever 800 may be hinged (hinge 802) at the wall 104 of the polymer holder 100. Then when the lever 800 is turned, an angled part of the lever 800 inside the polymer holder 100 is lifted toward the first aperture 108 whereby pushing also the biosignal processing device 102 toward the first aperture 108.

In an embodiment an example of which is shown in Figure 9, there may be a seal 900 around the electrically conductive contact structure 110. Although the electrically conductive contact structure 110 is a male connector in Figure 9, a similar sealing may be applied to a female connector. When the counterpart 116 is in contact with the electrically conductive contact structure 110, the coupling may be dust-proof and/or water-proof. The seal 900 may also increase the friction between the polymer holder 100 and the biosignal processing device 102.

An electrode system may comprises the polymer holder 100, the electrically conductive contact structure 110, the electrodes 112, and the biosignal processing device 102 which have already been disclosed above.

In an embodiment an example of which is shown in Figure 10, the biosignal processing device 102 may comprise one or more processors 142, and one or more memories 144 that may include a computer program code. The one or more memories 144 and the computer program code may, with the one or more processors 142, cause the electrode system at least to process the biosignal received from the human or animal.

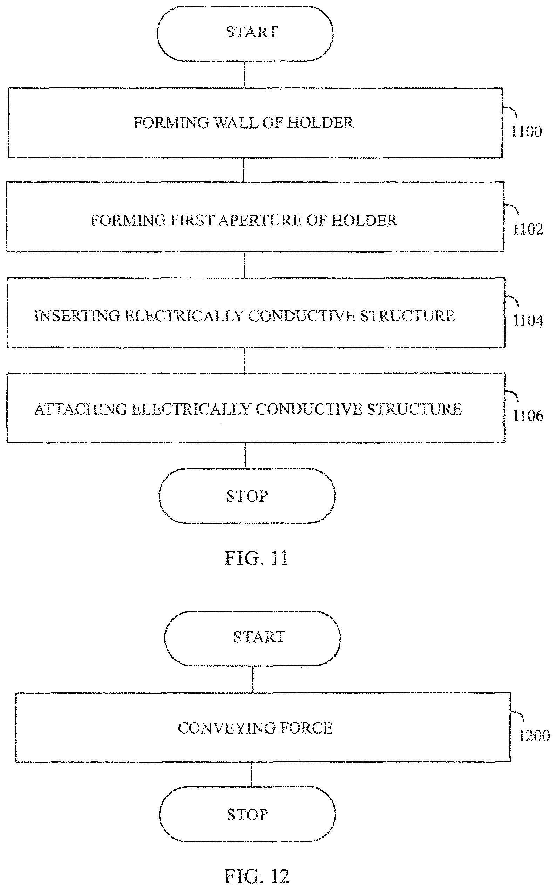

Figure 11 illustrates an example of a flow chart of a manufacturing method of a polymer holder 100. In step 1100, a wall 104 of the polymer holder 100 is formed in a shape of a pocket 106 that follows an outer contour of the biosignal processing device 102. In step 1102, a first aperture 108 is formed in the polymer holder 100 for allowing an insert of the biosignal processing device 102 into the pocket 106 and a removal of the biosignal processing device 102 from the pocket 106. In step 1104, an electrically conductive contact structure 110, which is electrically wired in contact with electrodes 112 that receive the at least one biosignal, is inserted at a rear section 114 of the polymer holder 100 opposite to the first aperture 108. In step 1106, the electrically conductive contact structure 110 is attached at least partly inside the wall 104, the electrically conductive contact structure 110 being for connection with a counterpart 116 of the biosignal processing device 102 in response to an insertion of the biosignal processing device 102 in the pocket 106.

In an embodiment of the manufacturing method, the electrically conductive contact structure 110 may be molded within an elastic material.

In the manufacturing method according to the invention, a mechanism 126 is formed at the rear section 114, the mechanism 126 being configured to cause a force, to the biosignal processing device 102, directed toward the first aperture 108 in response to pressure against the polymer holder 100 at the rear section 114.

Figure 12 illustrates an example of a flow chart of a handling method of a polymer holder 100. In step 1200, a force is conveyed to a biosignal processing device 102 by a mechanism 126 that is in contact with the biosignal processing device 102 at a rear section 114 of the polymer holder 100, the force moving the biosignal processing device 102 in response to a pressure against the polymer holder 102 at the rear section 114 toward a first aperture 108.

It will be obvious to a person skilled in the art that, as technology advances, the inventive concept can be implemented in various ways. The invention and its embodiments are not limited to the example embodiments described above but may vary within the scope of the claims.

## Claims

1. A polymer holder for a biosignal processing device, wherein a wall (140) of the polymer holder (100) is configured to form a pocket (106), the wall (140) being configured to follow an outer contour of the biosignal processing device (102), the polymer holder (100) comprising :
- a first aperture (108) for inserting the biosignal processing device (102) into the pocket (106) and removing the biosignal processing device (102) from the pocket (106);
- an electrically conductive contact structure (110), which is configured to be in a wired electric contact (122) with electrodes (112) configured to receive a biosignal, inside the polymer holder (100) at a rear section (114) opposite to the first aperture (108), and the electrically conductive contact structure (110) is at least partly attached inside the wall (140), the electrically conductive contact structure (110) being configured to electrically connect with a counterpart (116) of the biosignal processing device (102) in response to an insert of the biosignal processing device (102) in the pocket (106), and
- at the rear section (114), a mechanism (126) configured to cause a force to the biosignal processing device (102) directed toward the first aperture (108) in response to pressure against the polymer holder (100) at the rear section (114), **characterized in that** said mechanism (126) comprises a piston (128) configured to move toward and fromward the first aperture (108), and at least one lever (130) hinged to the piston (128) at one end and to the polymer holder (100) at another end;
wherein the lever (130) is configured to move the piston (128) toward the first aperture (108) in response to the pressure; and
wherein the piston (128) is configured to move the biosignal processing device (102) toward the first aperture (108).

2. The polymer holder of claim 1, wherein the electrically conductive contact structure (110) comprises a male connector partly inside the pocket (106), and the male connector being configured to connect with a female connector of the biosignal processing device (102) in response to the insert of the biosignal processing device (102) in the pocket (106).

3. The polymer holder of claim 1, wherein the electrically conductive contact structure (110) comprises a female connector, and the female connector is configured to connect with a male connector of the biosignal processing device (102) in response to the insert of the biosignal processing device (102) in the pocket (106).

4. The polymer holder of claim 1, wherein the electrically conductive contact structure (110) is partly within a cover (118) of an elastic material.

5. The polymer holder of claim 1, wherein the polymer holder has a second aperture (120) at the rear section (114), a normal (N2) of the second aperture (120) being directed toward a direction perpendicular to a normal (N1) of the first aperture (108).

6. The polymer holder of claim 1, wherein the polymer holder (100) has a rail (136) extending longitudinally in a direction from the rear section (114) to the first aperture (108), and the polymer holder (100) has a slit (138) into which the rail (136) is matched for guiding the movement of the piston (128) toward the first aperture (108).

7. An electrode system, **characterized in that** the electrode system comprises the polymer holder (100) of claim 1, electrodes (112) configured to receive a biosignal, and a biosignal processing device (102).

8. The electrode system of claim 7, wherein
the biosignal processing device (112) comprises:
one or more processors (142); and
one or more memories (144) including computer program code;
the one or more memories (144) and the computer program code configured to, with the one or more processors (142), cause the electrode system at least to process the biosignal received from a human or animal.

9. A manufacturing method of a polymer holder for a biosignal ssing device, comprising the steps of:
forming (1100) a wall (140) of the polymer holder (100) in a shape of a pocket (106) that follows an outer contour of the biosignal processing device (102);
forming (1102) a first aperture (108) in the polymer holder (100) for allowing an insert of the biosignal processing device (102) into the pocket (106) and a removal of the biosignal processing device (102) from the pocket (106);
inserting (1104) an electrically conductive contact structure (110), which is electrically wired in contact with electrodes (112) that receive a biosignal, at a rear section (114) of the polymer holder (100) opposite to the first aperture (108);
attaching (1106) the electrically conductive contact structure (110) at least partly inside the wall (104), the electrically conductive contact structure (110) being for a connection with a counterpart (116) of the biosignal processing device (102) in response to an insertion of the biosignal processing device(102) in the pocket (106); and
forming, at the rear section (114) of the polymer holder (100), a mechanism (126) configured to cause a force to the biosignal processing device (102) directed toward the first aperture (108) in response to pressure against the polymer holder (100) at the rear section (114), said mechanism comprising a piston (128) that is configured to move toward and fromward the first aperture (108), and at least one lever (130) hinged to the piston (128) at one end and to the polymer holder (100) at another end, the lever (130) being configured to move the piston (128) toward the first aperture (108) in response to the pressure, and the piston (128) is configured to move the biosignal processing device (102) toward the first aperture (108).

10. The method of claim 9, the method further comprising molding the electrically conductive contact structure (110) within a cover (118) of an elastic material.

11. A handling method of a polymer holder for a biosignal processing device according to claim 1, the wall (140) of the holder configured to form a pocket following an outer contour of the biosignal processing device (102);
the handling method comprising
conveying (1200), by a mechanism (126) that comprises a piston (128) and at least one lever (130) hinged to the piston (128) at one end and to the polymer holder (100) at another end, the mechanism (126 being in contact with the biosignal processing device (102) at a rear section (114) of the polymer holder (100), a force that moves the biosignal processing devise (102) in response to pressure of the polymer holder (100) at the rear section (114) toward a first aperture (108) such that
the piston (128) is movable toward and fromward the first aperture (108), the lever (130) moves the piston (128) toward the first aperture (108) in response to the pressure, and the piston (128) moves the biosignal processing device (102) toward the first aperture (108).

## Patentansprüche

1. Polymerhalter für ein Biosignalverarbeitungsgerät, wobei eine Wand (140) des Polymerhalters (100) dazu ausgestaltet ist, eine Tasche (106) zu bilden, wobei die Wand (140) dazu ausgestaltet ist, einem äußeren Umriss des Biosignalverarbeitungsgeräts (102) zu folgen, wobei der Polymerhalter (100) umfasst:
- eine erste Öffnung (108) zum Einführen des Biosignalverarbeitungsgeräts (102) in die Tasche (106) und Entfernen des Biosignalverarbeitungsgeräts (102) aus der Tasche (106);
- eine elektrisch leitfähige Kontaktstruktur (110), die dazu ausgestaltet ist, in einem drahtgebundenen elektrischen Kontakt (122) mit Elektroden (112) zu stehen, die dazu ausgestaltet sind, ein Biosignal zu empfangen, im Inneren des Polymerhalters (100) an einem hinteren Abschnitt (114) entgegengesetzt zu der ersten Öffnung (108), und die elektrisch leitfähige Kontaktstruktur (110) zumindest teilweise im Inneren der Wand (140) befestigt ist, wobei die elektrisch leitfähige Kontaktstruktur (110) dazu ausgestaltet ist, sich als Reaktion auf ein Einführen des Biosignalverarbeitungsgeräts (102) in der Tasche (106) elektrisch mit einem Gegenstück (116) des Biosignalverarbeitungsgeräts (102) zu verbinden, und
- am hinteren Abschnitt (114), einen Mechanismus (126), der dazu ausgestaltet ist, zu bewirken, dass eine Kraft auf das Biosignalverarbeitungsgerät (102) als Reaktion auf Druck gegen den Polymerhalter (100) am hinteren Abschnitt (114) hin zur ersten Öffnung (108) gerichtet wird, **dadurch gekennzeichnet, dass** der Mechanismus (126) einen Kolben (128), der dazu ausgestaltet ist, sich hin zur ersten Öffnung (108) und von ihr weg zu bewegen, und mindestens einen Hebel (130) umfasst, der an einem Ende an dem Kolben (128) und am anderen Ende an den Polymerhalter (100) gelenkig belagert ist;
wobei der Hebel (130) dazu ausgestaltet ist, den Kolben (128) als Reaktion auf den Druck hin zur ersten Öffnung (108) zu bewegen; und
wobei der Kolben (128) dazu ausgestaltet ist, das Biosignalverarbeitungsgerät (102) hin zur ersten Öffnung (108) zu bewegen.

2. Polymerhalter nach Anspruch 1, wobei die elektrisch leitfähige Kontaktstruktur (110) einen aufgenommenen Verbinder teilweise im Inneren der Tasche (106) umfasst, und der aufgenommene Verbinder dazu ausgestaltet ist, sich als Reaktion auf das Einführen des Biosignalverarbeitungsgeräts (102) in der Tasche (106) mit einem aufnehmenden Verbinder des Biosignalverarbeitungsgeräts (102) zu verbinden.

3. Polymerhalter nach Anspruch 1, wobei die elektrisch leitfähige Kontaktstruktur (110) einen aufnehmenden Verbinder umfasst, und der aufnehmende Verbinder dazu ausgestaltet ist, sich als Reaktion auf das Einführen des Biosignalverarbeitungsgeräts (102) in der Tasche (106) mit einem aufgenommenen Verbinder des Biosignalverarbeitungsgeräts (102) zu verbinden.

4. Polymerhalter nach Anspruch 1, wobei die elektrisch leitfähige Kontaktstruktur (110) sich teilweise innerhalb einer Abdeckung (118) aus einem elastischen Material befindet.

5. Polymerhalter nach Anspruch 1, wobei der Polymerhalter eine zweite Öffnung (120) am hinteren Abschnitt (114) aufweist, wobei eine Normale (N2) der zweiten Öffnung (120) hin zu einer Richtung senkrecht zu einer Normalen (N1) der ersten Öffnung (108) gerichtet ist.

6. Polymerhalter nach Anspruch 1, wobei der Polymerhalter (100) eine Schiene (136) aufweist, die sich längs in einer Richtung von dem hinteren Abschnitt (114) zur ersten Öffnung (108) erstreckt, und der Polymerhalter (100) einen Schlitz (138) aufweist, in den die Schiene (136) zum Führen der Bewegung des Kolbens (128) hin zur ersten Öffnung (108) eingepasst ist.

7. Elektrodensystem, **dadurch gekennzeichnet, dass** das Elektrodensystem den Polymerhalter (100) nach Anspruch 1, Elektroden (112), die dazu ausgestaltet sind, ein Biosignal zu empfangen, und ein Biosignalverarbeitungsgerät (102) umfasst.

8. Elektrodensystem nach Anspruch 7, wobei
das Biosignalverarbeitungsgerät (112) umfasst:
einen oder mehrere Prozessoren (142); und
einen oder mehrere Speicher (144), die Computerprogrammcode umfassen;
wobei der eine oder die mehreren Speicher (144) und der Computerprogrammcode dazu ausgestaltet sind, mit dem einen oder den mehreren Prozessoren (142) zu bewirken, dass das Elektrodensystem zumindest das Biosignal verarbeitet, das von einem Menschen oder Tier empfangen wird.

9. Verfahren zur Herstellung eines Polymerhalters für ein Biosignalverarbeitungsgerät, das die folgenden Schritte umfasst:
Bilden (1100) einer Wand (140) des Polymerhalters (100) in einer Form einer Tasche (106), die einem äußeren Umriss des Biosignalverarbeitungsgeräts (102) folgt;
Bilden (1102) einer ersten Öffnung (108) in dem Polymerhalter (100) zum Erlauben eines Einführens des Biosignalverarbeitungsgeräts (102) in die Tasche (106) und eines Entfernens des Biosignalverarbeitungsgeräts (102) aus der Tasche (106);
Einführen (1104) einer elektrisch leitfähigen Kontaktstruktur (110), die elektrisch in Kontakt mit Elektroden (112) verdrahtet ist, die ein Biosignal empfangen, an einem hinteren Abschnitt (114) des Polymerhalters (100) entgegengesetzt zur ersten Öffnung (108);
Anbringen (1106) der elektrisch leitfähigen Kontaktstruktur (110) zumindest teilweise im Inneren der Wand (104), wobei die elektrisch leitfähige Kontaktstruktur (110) für eine Verbindung mit einem Gegenstück (116) des Biosignalverarbeitungsgeräts (102) als Reaktion auf eine Einführung des Biosignalverarbeitungsgeräts (102) in der Tasche (106) bestimmt ist; und
Bilden, am hinteren Abschnitt (114) des Polymerhalters (100), eines Mechanismus (126), der dazu ausgestaltet ist, als Reaktion auf Druck gegen den Polymerhalter (100) am hinteren Abschnitt (114) eine Kraft auf das Biosignalverarbeitungsgerät (102) zu bewirken, die hin zur ersten Öffnung (108) gerichtet ist, wobei der Mechanismus einen Kolben (128), der dazu ausgestaltet ist, sich hin zur ersten Öffnung (108) und davon weg zu bewegen, und mindestens einen Hebel (130) umfasst, der an einem Ende am Kolben (128) und am anderen Ende am Polymerhalter (100) gelenkig gelagert ist, wobei der Hebel (130) dazu ausgestaltet ist, als Reaktion auf den Druck den Kolben (128) hin zur ersten Öffnung (108) zu bewegen, und der Kolben (128) dazu ausgestaltet ist, das Biosignalverarbeitungsgerät (102) hin zur ersten Öffnung (108) zu bewegen.

10. Verfahren nach Anspruch 9, wobei das Verfahren ferner das Formen der elektrisch leitfähigen Kontaktstruktur (110) innerhalb einer Abdeckung (118) aus einem elastischen Material umfasst.

11. Verfahren zur Handhabung eines Polymerhalters für ein Biosignalverarbeitungsgerät nach Anspruch 1, wobei die Wand (140) des Halters dazu ausgestaltet ist, eine Tasche zu bilden, die einem äußeren Umriss des Biosignalverarbeitungsgeräts (102) folgt;
wobei das Handhabungsverfahren umfasst:
Befördern (1200), durch einen Mechanismus (126), der einen Kolben (128) und mindestens einen Hebel (130) umfasst, der an einem Ende am Kolben (128) und am anderen Ende am Polymerhalter (100) gelenkig gelagert ist, wobei der Mechanismus (126) an einem hinteren Abschnitt (114) des Polymerhalters (100) mit dem Biosignalverarbeitungsgerät (102) in Kontakt ist, einer Kraft, welche das Biosignalverarbeitungsgerät (102) als Reaktion auf Druck des Polymerhalters (100) am hinteren Abschnitt (114) hin zu einer ersten Öffnung (108) bewegt, derart dass
der Kolben (128) hin zur ersten Öffnung (108) und davon weg beweglich ist, der Hebel (130) den Kolben (128) als Reaktion auf den Druck hin zur ersten Öffnung (108) bewegt und der Kolben (128) das Biosignalverarbeitungsgerät (102) hin zur ersten Öffnung (108) bewegt.

## Revendications

1. Support polymère pour un dispositif de traitement de signal biologique, dans lequel une paroi (140) du support polymère (100) est configurée pour former une poche (106), la paroi (140) étant configurée pour suivre un contour extérieur du dispositif de traitement de signal biologique (102), le support de polymère (100) comprenant :
une première ouverture (108) pour insérer le dispositif de traitement de signal biologique (102) dans la poche (106) et sortir le dispositif de traitement de signal biologique (102) de la poche (106) ;
une structure de contact électriquement conductrice (110), qui est configurée pour être en contact électrique câblé (122) avec des électrodes (112) configurées pour recevoir un signal biologique, à l'intérieur du support polymère (100) au niveau d'une section arrière (114) opposée à la première ouverture (108), et laquelle structure de contact électriquement conductrice (110) est au moins partiellement attachée à l'intérieur de la paroi (140), la structure de contact électriquement conductrice (110) étant configurée pour se connecter électriquement à un homologue (116) du dispositif de traitement de signal biologique (102) en réponse à une insertion du dispositif de traitement de signal biologique (102) dans la poche (106), et
au niveau de la section arrière (114), un mécanisme (126) configuré pour appliquer une force sur le dispositif de traitement de signal biologique (102) dirigée vers la première ouverture (108) en réponse à une pression contre le support polymère (100) au niveau de la section arrière (114),
**caractérisé en ce que** ledit mécanisme (126) comprend un piston (128) configuré pour se déplacer vers et depuis la première ouverture (108), et au moins un levier (130) articulé au piston (128) au niveau d'une extrémité et au support polymère (100) au niveau d'une autre extrémité ;
dans lequel le levier (130) est configuré pour déplacer le piston (128) vers la première ouverture (108) en réponse à la pression ; et
dans lequel le piston (128) est configuré pour déplacer le dispositif de traitement de signal biologique (102) vers la première ouverture (108).

2. Support polymère selon la revendication 1, dans lequel la structure de contact électriquement conductrice (110) comprend un connecteur mâle partiellement à l'intérieur de la poche (106), le connecteur mâle étant configuré pour se connecter à un connecteur femelle du dispositif de traitement de signal biologique (102) en réponse à l'insertion du dispositif de traitement de signal biologique (102) dans la poche (106).

3. Support polymère selon la revendication 1, dans lequel la structure de contact électriquement conductrice (110) comprend un connecteur femelle, et le connecteur femelle est configuré pour se connecter à un connecteur mâle du dispositif de traitement de signal biologique (102) en réponse à l'insertion du dispositif de traitement de signal biologique (102) dans la poche (106).

4. Support polymère selon la revendication 1, dans lequel la structure de contact électriquement conductrice (110) est partiellement à l'intérieur d'un couvercle (118) en un matériau élastique.

5. Support polymère selon la revendication 1, lequel support polymère a une deuxième ouverture (120) au niveau de la section arrière (114), une normale (N2) de la deuxième ouverture (120) étant dirigée vers une direction perpendiculaire à une normale (N1) de la première ouverture (108).

6. Support polymère selon la revendication 1, lequel support polymère (100) a un rail (136) s'étendant longitudinalement dans une direction allant de la section arrière (114) à la première ouverture (108), et lequel support polymère (100) a une fente (138) dans laquelle le rail (136) est apparié pour guider le mouvement du piston vers la première ouverture (108).

7. Système d'électrodes, **caractérisé en ce que** le système d'électrodes comprend le support polymère (100) de la revendication 1, des électrodes (112) configurées pour recevoir un signal biologique, et un dispositif de traitement de signal biologique (102).

8. Système d'électrodes selon la revendication 7, dans lequel le dispositif de traitement de signal biologique (112) comprend :
un ou plusieurs processeurs (142) ; et
une ou plusieurs mémoires (144) contenant un code de programme informatique ;
la ou les mémoires (144) et le code de programme informatique étant configuré pour, avec le ou les processeurs (142) amener le système d'électrodes au moins à traiter le signal biologique reçu depuis un humain ou un animal.

9. Procédé de fabrication d'un support polymère pour un dispositif de traitement de signal biologique, comprenant les étapes de :
formation (1100) d'une paroi (140) du support polymère (100) sous la forme d'une poche (106) qui suit le contour extérieur du dispositif de traitement de signal biologique (102) ;
formation (1102) d'une première ouverture (108) dans le support polymère (100) pour permettre l'insertion du dispositif de traitement de signal biologique (102) dans la poche et le retrait du dispositif de de traitement de signal biologique (102) hors de la poche (106) ;
insertion (1104) d'une structure de contact électriquement conductrice (110), qui est électriquement câblée en contact avec des électrodes (112) qui reçoivent un signal biologique, au niveau d'une section arrière (114) du support polymère (100) opposée à la première ouverture (108) ;
attachement (1106) de la structure de contact électriquement conductrice (110) au moins partiellement à l'intérieur de la paroi (104), la structure de contact électriquement conductrice (110) étant pour une connexion à un homologue (116) du dispositif de traitement de signal biologique (102) en réponse à l'insertion du dispositif de traitement de signal biologique (102) dans la poche (106) ; et
formation, au niveau de la section arrière (114) du support polymère (100), d'un mécanisme (126) configuré pour appliquer une force sur le dispositif de traitement de signal biologique (102) dirigée vers la première ouverture (108) en réponse à une pression contre le support polymère (100) au niveau de la section arrière (114), ledit mécanisme comprenant un piston (128) configuré pour se déplacer vers et depuis la première ouverture (108), et au moins un levier (130) articulé au piston (128) au niveau d'une extrémité et au support polymère (100) au niveau d'une autre extrémité, le levier (130) étant configuré pour déplacer le piston (128) vers la première ouverture (108) en réponse à la pression, et le piston (128) étant configuré pour déplacer le dispositif de traitement de signal biologique (102) vers la première ouverture (108).

10. Procédé selon la revendication 9, le procédé comprenant en outre le moulage de la structure de contact électriquement conductrice (110) à l'intérieur d'un couvercle (118) en un matériau élastique.

11. Procédé de manipulation d'un support polymère pour un dispositif de traitement de signal biologique selon la revendication 1, la paroi (140) du support étant configurée pour former une poche suivant le contour extérieur du dispositif de traitement de signal biologique (102) ;
le procédé de manipulation comprenant
le convoyage (1200), par un mécanisme (126) qui comprend un piston (128) et au moins un levier (130) articulé au piston (128) au niveau d'une extrémité et au support polymère (100) au niveau d'une autre extrémité, le mécanisme (126) étant en contact avec le dispositif de traitement de signal biologique au niveau d'une section arrière (114) du support polymère (100), d'une force qui déplace le dispositif de traitement de signal biologique (102) en réponse à la pression du support polymère (100) au niveau de la section arrière (114) vers une première ouverture (108) de telle sorte que
le piston (128) soit mobile vers et depuis la première ouverture (108), le levier (130) déplace le piston (128) vers la première ouverture (108) en réponse à la pression, et le piston (128) déplace le dispositif de traitement de signal biologique (102) vers la première ouverture (108).
